# EUROPEAN PATENT APPLICATION

(11) **EP 2 740 473 A1**
(43) Date of publication of application: **11.06.2014**
(21) Application number: 13196003.1
(22) Date of filing: 06.12.2013
(51) Int. Cl.: A61K 31/05, A61K 31/14, A61K 31/202, A61P 1/16

(54) **Composition for the prevention and the treatment of hepatic steatosis and/or cystic fibrosis**

(30) Priority: 06.12.2012 IT MI20122088
(71) Applicant: DMF Dietetic Metabolic Food S.R.L., 20812 Limbiate (IT)
(72) Inventor: Vicentini, Ultimo Vito, 20812 Limbiate (IT)
(74) Representative: Coppo, Alessandro

(57) **Abstract**

The present inventions relates, in one aspect, to a composition comprising docosahexaenoic acid and choline in combination as active ingredients, optionally an active ingredient having cellular antioxidant activity, in particular vitamin E, and an edible vehicle. The composition of the invention finds application in the prevention and treatment of cystic fibrosis and/or hepatic steatosis, in particular of of non-alcoholic type.

## Description

### FIELD OF THE INVENTION

The present invention concerns a composition for the prevention and/or the treatment of hepatic steatosis and/or cystic fibrosis.

The present invention originates in the field of pharmaceuticals, dietary or food supplements, particularly intended for special medical purposes.

### STATE OF THE ART

Hepatic steatosis, also referred to as fatty liver disease, is a cellular pathology linked to the intracellular accumulation of triglycerides and lipids at liver tissue level, which involves a series of damages to this organ, until reaching the necrosis of the tissues of which it is formed. Generally, hepatic steatosis is characterized by the presence of lipids in the liver, in a quantity greater than 5% of its weight. Hepatic steatosis can be classified on the basis of the etiology into three main groups which include a) steatosis induced by increased fat intake, b) steatosis induced by reduced fats disposal, and c) steatosis induced by increased endogenous synthesis of fatty acids.

In the first of the three groups, the cause is often attributable to a high fat diet or to increased mobilization of non-esterified fatty acids (NEFA). The latter, in turn, can be determined by many factors including the intake of hormones, excessive quantities of caffeine, or even by a prolonged fasting, or other diseases where insulin deficiency occurs, with a consequent increase in lipolysis and circulating NEFA, as in case of diabetes mellitus or type I glycogenosis.

Among the causes of the steatosis of group b) there are: a low-protein diet, a decreased synthesis of apolipoproteins due to a deficiency of choline or vitamin B12, or an excess of vitamin PP or hypoxia.

The causes of steatosis by increased synthesis (group c) comprise accumulation of lipids by endogenous synthesis from acetate.

Clinically relevant examples are the steatosis caused by barbiturates and those by alcohol abuse in which the ethanol is metabolized to acetaldehyde, first, and then to acetate.

Despite a multiplicity of possible causes, hepatic steatosis can be considered a disease that usually occurs in people who have an excessive consumption of alcohol and/or suffering from obesity.

From a morphological point of view, it is however difficult to distinguish between the non- alcoholic hepatic steatosis and the one from alcohol, being both united by changes in the micro- and macro-vesicular fat, in various stages of generation. At cellular level, in fact, the steatosis describes a process in which an abnormal retention of lipids in the cell occurs. This reflects a malfunction of the processes physiologically responsible for the synthesis and the disposal of triglycerides. The fat in excess is accumulated in vesicles that displace the cytoplasm. When the vesicles reach such dimensions as to deform the nucleus of the cell, the pathology is defined as macrovesicular steatosis; otherwise the disease is referred to as microvesicular steatosis. The excessive accumulation of fat in the cells of the liver tissue is accompanied by a progressive inflammation called steatohepatitis.

Since the intake of alcohol leads then to a significant contribution in the development of the disease, it is possible to discriminate between a form of alcoholic steatosis in which alcohol is the main etiological cause, and a nonalcoholic hepatic steatosis or Nonalcoholic Fatty Liver Disease (so called NAFLD) not linked to alcohol intake. This second pathological condition is found in obese subjects with an incidence of 57-74% and it is often associated with type II diabetes.

In ultrasound examination, many subjects show signs of hepatic steatosis without obvious clinical manifestations. Typically, insulin-resistant subjects have a strong lipolytic activity, which leads to an overload of β-oxidation and contributes to steatosis.

The excess of fatty acids involves their lipoperoxidation that can trigger chronic inflammation (steatohepatitis) which ends with the development of cirrhosis and the impairment of the liver function.

The treatment and the prevention of steatosis are then based on maintaining a proper diet, the gradual decline in the body weight and a regular aerobic physical activity exercise.

To date, there are no standardized medical or surgical therapies for the treatment of hepatic steatosis.

Generally, the drugs that are administered for the treatment of this disease are antioxidant, insulin-sensitizing, lipid-lowering and cytoprotective supplements.

To date, the available drug and dietary-nutritional therapies have not been effective in reducing, in a satisfactory manner, the incidence of this disease.

In a completely parallel, there are no pharmaceutical or dietary products currently available on the market able to provide an adequate therapeutic response to the cases of cystic fibrosis (CF).

Cystic fibrosis, also known as mucoviscidosis, or fibrocystic disease of the pancreas, is a fatal inherited disease more common in the Caucasian population.

It is an autosomal recessive disorder caused by a mutation in the gene CFTCR (Cystic Fibrosis Transmembrane Conductance Regulator). This gene encodes a protein of 1480 amino acids located on the cell membrane of epithelial cells, which performs the function of carrying the chlorine through the cell membranes in the apical membrane of epithelial cells in different districts of the human body, such as the airways, intestine, pancreas, sweat glands, salivary glands and the vas deferens. The ionic imbalance resulting in the onset of the disease is caused by an alteration of the secretion of chlorine ions by the epithelial cells, which determines an increased reabsorption of water and sodium.

This disease is characterized, therefore, by an abnormality in chloride transport in the cell membrane of the glands with external secretion. Consequently, these glands secrete a thick, sticky mucus, substantially non-flowing. In the concerned organs, the mucous secretions, being abnormally slippery, determine an obstruction of the main ducts, causing the onset of most of the typical clinical manifestations of the disease, such as recurrent pulmonary infections, pancreatic insufficiency, steatorrhea, states of malnutrition, cirrhosis of the liver, intestinal obstruction and male infertility.

The therapeutic scheme involves taking a dietary regimen, a nutritional-digestive treatment, a respiratory physiotherapy with removal of bronchial secretions, a continuous, or in cycles, antibiotic therapy of respiratory infections, the treatment of the upper airways diseases and a medical-surgical therapy of the complications, with possible lung transplant.

In particular, the dietary regimen should be hypercaloric, rich in salts and in essential vitamins such as A, D, E and K. In addition, pancreatic enzymes should be administered during meals.

Also in this case, the pharmaceuticals or dietary products currently available on the market do not provide an adequate therapeutic response.

Currently, there is therefore the need for new pharmaceuticals or dietary products suitable to prevent, slow down and/or treat cases of hepatic steatosis or cystic fibrosis.

Therefore, one of the purposes of the present invention is to provide a composition capable of improving the clinical picture in patients with hepatic steatosis, in particular of non-alcoholic type (NAFLD), or cystic fibrosis.

Another object of the invention is to provide a food or dietary supplement that slow down the progression of cystic fibrosis or hepatic steatosis, in particular of non-alcoholic type (NAFLD), and reduces the pain associated with them.

### SUMMARY

The inventors of the present invention have found that the combination of docosahexaenoic acid with choline determines, at cellular level, a synergistic antioxidant effect that reduces the inflammatory and the oxidative component, both present in hepatic steatosis and cystic fibrosis, reducing the symptoms related to these two diseases.

In view of the purposes set forth above, the present invention relates, in a first aspect, to a composition comprising docosahexaenoic acid and choline as active ingredients, provided with a cellular antioxidant activity, and an edible vehicle. According to some embodiments of the invention, the composition further comprises an antioxidant substance.

In some preferred embodiments of the invention, the antioxidant substance comprises vitamin E.

The composition of the invention is specifically indicated for use in the prevention and/or treatment of hepatic steatosis, in particular of non-alcoholic type (NAFLD) and/or of cystic fibrosis, and the symptoms associated therewith.

According to a second aspect, the present invention relates to a composition comprising docosahexaenoic acid and choline as active ingredients, provided with a cellular antioxidant activity, and an edible vehicle for use in the treatment and/or prevention of hepatic steatosis, in particular of non-alcoholic type (NAFLD). According to the third aspect of the invention, a composition is provided comprising docosahexaenoic acid and choline as active ingredients, provided with a cellular antioxidant activity, and an edible vehicle for use in the treatment and/or prevention of cystic fibrosis.

According to some embodiments, the composition of the invention is in the form of a pharmaceutical composition comprising a pharmaceutically acceptable carrier. According to certain embodiments, the composition of the invention is in the form of a food supplement or a dietary product comprising an edible vehicle.

### DETAILED DESCRIPTION OF THE INVENTION

According to some aspects of the invention, the applicant has found that, by combining or associating a specific essential fatty acid of the omega-3 series with choline, a synergistic antioxidant effect is obtained which reduces the symptoms associated with certain diseases that are united by an excessive cellular oxidative activity, such as cystic fibrosis and hepatic steatosis, particularly of non-alcoholic type (NAFLD).

Accordingly, the present invention provides, in a first aspect, a composition comprising docosahexaenoic acid and choline as the active ingredients with antioxidant activity, both in an amount effective in reducing the cellular oxidative phenomena, and an edible vehicle.

One of the components of the composition of the invention is docosahexaenoic acid (22:6 n-3 or DHA), an essential fatty acid of the omega-3 series (PUFA). Typically, the chemical structure of this acid provides a chain comprising 22 carbon atoms, along which six cis double bonds are distributed, the first of these links the third and fourth carbon from the methyl portion (terminal), from which the name of ω3.

According to some embodiments, the composition of the invention comprises DHA in an amount ranging from 0.01% to 85%, more preferably from 10 to 65%, even more preferably from 60 to 65% by weight with respect to the total weight of the composition.

In the context of the invention, the algae may represent a suitable natural source of docosahexaenoic acid (DHA), or oils containing DHA, indicated for the formulation of the composition of the invention.

For example, the docosahexaenoic acid used in the invention can be obtained in the form of oil from an alga, in particular belonging to the genus Ulkenia or Schizochytrium.

Specifically, the oil of Ulkenia rich in DHA is a triacylglycerol oil derived from the species Ulkenia, a marine protist, having a total fatty acid composition from 38 to 50% of DHA. The remaining DHA oil fatty acids of Ulkenia comprise mainly the saturated palmitic acid (16:O) (28 - 37%) and in a smaller quantity, generally from 8 to 14% by weight of the fatty acid omega 6, docosapentaenoic acid (DPA). Typically, the oil of Ulkenia rich in DHA is produced through a multi-step fermentation and, preferably, through a refining process that uses a genetically pure and stable crop of the marine protist Ulkenia sp.

In particular, the DHA oil of Ulkenia is extracted from a dry biomass through a pressing step, or directly from a wet biomass through cell rupture and oil separation. Optionally, a solvent based process of extraction can be used which employs food grade solvents and processing aids. The crude olive oil extract can be further refined through conventional treatments in the field of edible oils, such as degumming, deacidification, bleaching, using substances suitable in the food field.

A method suitable for obtaining DHA oil of Ulkenia is described in GRAS Notice (GRN) No. 319, of FDA published on the website:
http://www.fda.gov/Food/FoodIngredientsPackaging/GenerallyRecognizedasSafe GRAS/GRASListings/default.htm, whose content is entirely incorporated herein.

The combination of DHA and choline determines an antioxidant effect and stimulation of cell metabolism particularly evident on the hepatocytes, reducing the liver inflammatory phenomena, thereby improving the symptoms of hepatic steatosis and/or cystic fibrosis.

Choline (2-hydroxy-N,N,N-trimethylethanammonium) is a saturated quaternary amine having formula [(CH3)3N+(CH2)2OH] X-, wherein X- represents a generic counterion of the ammonium salt, such as chloride anion, hydroxyl or tartrate. Choline is a constituent of the phospholipids that make up the cell membrane and the neurotransmitter acetylcholine.

According to some embodiments, the composition comprises choline in a quantity from 0.01 to 30% by weight, preferably from 1 to 10% by weight, based on the total weight of the composition.

The concomitant presence of DHA and choline in the formulation of the composition of the invention substantially improves the cell metabolism by improving the general condition of the organism subject to cystic fibrosis or fatty liver disease.

According to some embodiments, the composition of the invention further comprises an antioxidant substance. In some preferred embodiments, this antioxidant substance includes vitamin E. In the context of the invention, vitamin E means to encompass one or more of tocotrienols α, β, γ or δ, tocopherols α, β, γ or δ, and mixtures thereof.

Vitamin E acts as an antioxidant by preventing the oxidation of polyunsaturated fatty acids, which determines the process of lipid peroxidation. This event, triggered by the action of free radicals, spreads through chain reactions that continue the process.

Vitamin E, in particular when associated with choline and DHA, substantially blocks this phenomenon donating an electron to peroxylipidic radicals, thereby making them less reactive and blocking, as a matter of fact, the lipid peroxidation. This redox reaction converts the vitamin E into an α-tocopheroxyl radical which is quite stable, thanks to the development of resonance phenomena, and which can react with vitamin C, or with glutathione, or with coenzyme Q10 to reform the α-tocopherol.

In the context of the invention, one or more antioxidant substances such as resveratrol, pycnogenols, catechins (green tea and its extracts) can be used in addition to or in substitution of vitamin E.

In particular, it was observed that administering to the body a composition based on DHA, choline and an antioxidant substance, more specifically vitamin E, the cell tropism of hepatocytes is improved, the oxidative cellular phenomena are reduced, decreasing the general state of inflammation of the body and, thereby, improving the symptoms linked to hepatic steatosis and/or cystic fibrosis.

The composition of the invention, based on the association of DHA and choline, and optionally an antioxidant substance such as vitamin E, finds then application in the treatment or prevention of hepatic steatosis and/or cystic fibrosis.

The composition of the invention may optionally contain one or more additional active ingredients or principles.

The composition of the invention contains, in addition to the association or combination of DHA and choline, one or more edible vehicles.

The term "vehicle" refers to an excipient, vehicle, diluent or adjuvant that may be present in the composition of the invention. Any vehicle and/or excipient suitable for the desired form of preparation for administration is contemplated in the use of the compounds or active ingredients described herein.

For the purposes of the present invention, the term "edible" means a substance similar to a food whose use in the pharmaceutical, dietary or food field has been approved by the Regulatory Authorities.

The composition of the invention comprises any composition or formulation obtained by mixing the association or combination of active ingredients according to one embodiment of the invention, and a pharmaceutically acceptable carrier. Such compositions are suitable for pharmaceutical, nutritional or dietary use in a human being or an animal.

The vehicle can assume a wide variety of forms depending on the type of preparation suitable for oral administration such as tablets, capsules, syrups. In preparing the compositions for the oral dosage form, one or more of the usual pharmaceutical or nutritional media may be used. In the case of liquid preparations, such as suspensions, solutions, elixirs, water, glycols, oils, alcohols, flavoring agents, preservatives, dyes, and the like can be used. In the case of preparations in solid form, such as tablets, capsules, syrups etc., vehicles such as starches, sugars, microcrystalline cellulose, diluents, granulating agents, lubricants, binders, disintegrants and similar may be used, being however the preparations in solid form preferred over those in liquid form.

According to some embodiments, the compounds of the present invention can be combined as the active ingredient in intimate admixture with a suitable edible vehicle and/or excipient according to conventional pharmaceutical or food techniques, or to processing techniques of dietary/nutritional products.

One route of administration of the composition of the invention is the oral route. The compositions may conveniently be presented in a unit dosage form and be prepared according to any of the methods well known in the art of pharmaceutical technique or food processing.

The pharmaceutical compositions may be in the form of tablets, pills, capsules, solutions, suspensions, emulsions in suitable controlled dosage forms.

If desired, the tablets may be coated by standard techniques in aqueous phase or not. In some embodiments, such compositions and preparations may contain at least 0.1 % of each of the active ingredients DHA and choline. The percentages of active ingredients in these compositions can vary for example from 1 to 60% by weight based on the weight of the unit. The quantity of active ingredient in these compositions is such as to obtain a prophylactic or therapeutic effect according to the invention.

Typically, tablets, capsules, pills and the like may also contain a binder such as gum tragacanth, acacia, corn starch, or gelatin; excipients such as dicalcium phosphate, a disintegrating agent such as potato starch, corn starch, alginic acid; a lubricant agent such as magnesium stearate; a sweetening agent such as sucrose, lactose or saccharin. When the dosage unit form is a capsule, it may contain, in addition to materials of the previously described type, a liquid carrier such as a fatty oil.

According to some embodiments, the composition of the invention further comprises one or more additional components, such as additives, bulking agents, fillers, stabilizers, emulsifiers, plasticizers, film-forming agents, humectants, thickeners, structuring agents.

Various other materials may be present as coatings or materials suitable to modify the physical form of the dosage unit.

For example, the tablets may be coated with shellac, sugar or both. In the cases of preparations such as syrups or elixirs, in addition to the association of active ingredients, sucrose as a sweetening agent, methyl and propyl-parabens as preservatives, one or more coloring and/or flavoring agents such as aromas of strawberry, cherry etc. may be present.

According to some embodiments, the composition may be a formulation with enteric coating in order to prevent breakage during the transit in the upper portion of the gastrointestinal tract.

According to some embodiments, in the composition of the invention, the combination of active ingredients is formulated in dosage units. The dosage unit may contain from 0.1 to 1000 mg of each of the active ingredients per dosage unit for daily administration.

In some embodiments, the effective quantities for formulation will depend on the severity of the disease, condition or pathology, the state of individual health and the therapeutic response to the association of active ingredients. In some embodiments, the dose to be administered varies from 0.001% by weight to approx. 60% by weight based on the weight of the formulation.

According to some embodiments, the composition of the invention is a nutritional or dietary product.

In the context of the invention, the term "synergism or synergistic activity" means an activity that is greater than the sum of the activities of the active ingredient when taken alone.

Typically, the synergism between drugs or supplements occurs when two active ingredients interact in ways that enhance or highlight one or more of their effects. The combination of DHA and choline according to the present invention determines the synergistic antioxidative action on hepatocytes that substantially slows the progression of the degenerative state of the liver tissue, typical of the hepatic steatosis and/or cystic fibrosis forms.

According to some embodiments, the composition of the invention may further contain a green tea extract.

In the context of the present invention, the term green tea extract means a product of conventional extraction of green tea leaves by a solvent. In some embodiments, the extraction is carried out using water as a solvent, heated to a temperature exceeding 50°C, and typically between 70 and 95°C, for a time between 2 minutes and one hour.

In some embodiments, the green tea extract can be prepared by extraction of dried leaves of green tea using water at 80°C for a period of approx. 15-25 minutes, followed by a second extraction with water placed at a temperature comprised between 90 and 97°C, for a period of time comprised between 5 and 15 minutes.

In some embodiments, the composition of the invention may further contain coenzyme Q10.

The present invention will now be described with reference to the following examples which are provided for illustrative purposes only and should not be intended as limiting the present invention.

### EXAMPLE 1

### INGREDIENTS:

Composition in the form of capsules or liquid capsules containing the following components:

Algal oil (Ulkenia sp., sunflower oil, tocopherols), Gelatin, Choline Bitartrate, Strength Agent: Glycerol, Natural vitamin E (D-alpha-tocopherol in sunflower oil and soybean), Thickener: Glycerol monostearate, Colloidal silica, Emulsifier: Sunflower lecithin. Anti-caking agent: E 555 (Potassium aluminum silicate), Dyes, E171, E172.

| Average Analysis | Per liquid capsule |
|---|---|
| Energetic Value | 7.318 Kcal / 30.287 KJ |
| Proteins | 0.236 g |
| Carbohydrates | 0.120 g |
| Of which: Sugars | 0 g |
| Fats | 0.655 g |
| Of which: Saturated | 0.149 g |
| Monounsaturated | 0.086 g |
| Polyunsaturated | 0.356 g |
| Of which: DHA | 0.250 g |
| Vitamin E | 26.000 mg-αTE |
| Choline bitartrate | 201.000 mg |

### EXAMPLE 2

Composition of the invention in the pharmaceutical form of a liquid capsule containing the following active ingredients expressed as percentages by weight referred to the total weight of the liquid capsule (1.272 g):
DHA: 19.65%
Colina 15.8%
Vit. E: 2.05%

## Claims

1. Composition for use in the treatment and/or prophylaxis of hepatic steatosis and/or cystic fibrosis **characterized in that** it comprises docosahexaenoic acid and choline in combination as active ingredients, optionally an active ingredient having cellular antioxidant activity, and an edible vehicle.

2. The composition for use according to claim 1, wherein the active ingredient having cellular antioxidant activity is selected from vitamin E, pycnogenols, phenols, catechins and mixtures thereof.

3. The composition for use according to claim 1 or 2, wherein the active ingredient having cellular antioxidant activity is vitamin E.

4. The composition for use according to any one of the claims 1-3, wherein said docosahexaenoic acid is contained in an oil preferably obtained from an alga of genus Ulkenia.

5. The composition for use according to any one of the claims 1-4 **characterized in that** it has the solid form of a tablet or capsule, a syrup or an emulsion.

6. The composition for use according to any one of the claims 1-5 **characterized in that** it is a dietary supplement or a dietetic product.

7. The composition for use according to any one of the claims 1-6 wherein DHA is present in an amount ranging from 0.001 to 85% by weight and choline in an amount from 0.001 to 30% by weight.

8. The composition for use according to any one of the claims 1-7 for the treatment and prevention of cystic fibrosis.

9. The composition for use according to any one of the claims 1-8 for the treatment and prevention of hepatic steatosis.

10. The composition for use according to claim 1 or 9 wherein the hepatic steatosis is of non-alcoholic type.
